# EUROPÄISCHE PATENTSCHRIFT

(11) **EP 0 725 773 B1**
(45) Veröffentlichungstag und Bekanntmachung des Hinweises auf die Patenterteilung: **10.09.1997**
(21) Anmeldenummer: 94930209.5
(22) Anmeldetag: 22.10.1994
(51) Int. Cl.: C07C 269/06, C07C 271/22, C07C 323/52

(54) **VERFAHREN ZUR HERSTELLUNG N-GESCHÜTZTER N-ALKYLIERTER AMINOSÄUREN**
PROCESS FOR PRODUCING N-PROTECTED N-ALKYLATED AMINO ACIDS
PROCEDES DE PREPARATION D'ACIDES AMINES A PROTECTION N ET ALKYLATION N

(30) Priorität: 02.11.1993 DE 4337331
(43) Veröffentlichungstag der Anmeldung: 14.08.1996
(73) Patentinhaber: BASF AKTIENGESELLSCHAFT, 67056 Ludwigshafen (DE)
(72) Erfinder: KNÜHL, Klaus, D-67117 Limburgerhof (DE); KARL, Ulrich, D-67061 Ludwigshafen (DE); MÜLLER, Stefan, D-67346 Speyer (DE); DE POTZOLLI, Bernd, D-67098 Bad Dürkheim (DE)
(86) Internationale Anmeldenummer: EP9403480
(87) Internationale Veröffentlichungsnummer: WO9512574

(56) Entgegenhaltungen:
- WO-A-90/06914
- JOURNAL OF THE CHEMICAL SOCIETY, CHEMICAL COMMUNICATIONS, Nr.6, 1972, LETCHWORTH GB Seiten 357 - 358 G. MARINO, L. VALENTE 'N-Methylation of peptides'
- CHEMICAL ABSTRACTS, vol. 112, no. 3, 15. Januar 1990, Columbus, Ohio, US; abstract no. 21273n, J. HLAVACEK ET AL. Seite 535 ; & COLLECT. CZECH. CHEM. COMMUN., Bd.53, Nr.11A, 1988 Seiten 2473 - 2494

## Beschreibung

Die vorliegende Erfindung betrifft ein neues Verfahren zur Herstellung N-geschützter N-alkylierter Aminosäuren. N-methylierte Aminosäuren stellen wichtige Bestandteile biologisch hochwirksamer Peptide dar. Beispiele für solche Peptide sind Cyclosporine (Angew. Chem. 1985,97,88) und Dolastatine (Nat. Prod. 1981,44,482).

Die Herstellung N-mono-methylierter Aminosäuren ohne die Verwendung von N-Schutzgruppen hat keine praktische Bedeutung erlangt. Die in der Literatur beschriebenen Verfahren erfordern den Einsatz teurer und schwierig zu handhabender Reagenzien (Methyliodid, Natriumhydrid) im Überschuß (Can. J. Chem. 1977,55,906). Außerdem ist die oftmals gleichzeitig auftretende Veresterung der Säurefunktion unerwünscht und störend (J. Org. Chem. 1970,35,1912).

Für den Einsatz in der Peptidchemie sind N-geschützte-N-methylierte Aminosäurederivate besonders vorteilhaft, da die methylierte Aminogruppe nicht reagieren kann und die Säurefunktion nicht freigesetzt werden muß. Das bislang praktikabelste Verfahren wurde von Runge (WO 90/06914) vorgestellt. Hierbei werden t-Butyloxycarbonyl geschützte Aminosäuren mit Methyliodid versetzt, und nach Zugabe von Kalium-tert.butanolat erfolgt die Methylierung. Jedoch sind bei dem in WO 90/06914 beschriebenen Verfahren sowohl die gewählte Schutzgruppe als auch das Methylierungsmittel für die Herstellung größerer Mengen N-geschützter-N-methylierter Aminosäuren nicht optimal. Verwendet man statt der t.-Butyloxycarbonyl- die Benzyloxycarbonyl-Schutzgruppe (im folgenden mit Z abgekürzt), so erhält man die gewünschte N-alkylierte Aminosäure nur in sehr mäßiger Ausbeute nebst vielen Nebenprodukten. Ersetzt man neben der Schutzgruppe auch das Methylierungsmittel (Dimethylsulfat statt Methyliodid), so wird kein Produkt mehr isoliert.

Überraschend wurde gefunden, daß durch Änderung der Reihenfolge der Reagenzienzugabe und durch eine Änderung des Alkylierungsmittels N-Z-geschützte N-Alkyl-aminosäuren in sehr guter Ausbeute in hoher optischer Reinheit hergestellt werden können.

Die vorliegende Erfindung betrifft ein Verfahren zur Herstellung N-geschützter N-alkylierter Aminosäuren der Formel I: 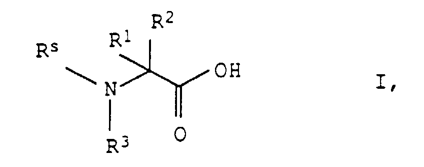 worin
- R^{s}: für eine in der Peptidsynthese übliche Schutzgruppe steht,
- R¹: die Seitenkette einer proteinogenen Aminosäure oder gegebenenfalls ein funktionelles Derivat derselben bedeutet,
- R²: für Wasserstoff, C₁₋₆-Alkyl, C₂₋₆-Alkenyl, C₂₋₆-Alkinyl oder gegebenenfalls C₁₋₄-alkylsubstituiertes Phenyl oder Benzyl steht und
- R³: Methyl oder Ethyl ist,
dadurch gekennzeichnet, daß man eine Verbindung der Formel II: 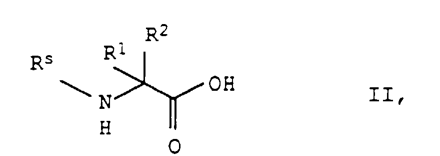 worin R¹, R² und R^{s} die oben angegebene Bedeutung haben, zu einer Lösung von Natrium- oder Kalium-tert.-butanolat in einem nichtprotischen organischen Lösungsmittel gibt und anschließend mit Dimethyl- oder Diethylsulfat versetzt.

Das Verfahren eignet sich sowohl zur Herstellung der racemischen Verbindungen als auch von enantionerenreinen Verbindungen.

In der Formel I haben die Substituenten vorzugsweise folgende Bedeutung:
- R¹: Der Rest eine proteinogene Aminosäure, insbesondere H, C₁₋₆-Alkyl- wie CH₃-, CH₃-CH₂-CH₂-, -C(CH₃)-CH₂-CH₃, -CH₂-CH(CH₃)₂-, C₆H₅-CH₂- und ganz besonders -CH(CH₃)₂

Als funktionelle Derivate von Resten proteinogener Aminosäuren seien für R¹ genannt:
-CH₂-C₆H₄O-C(CH₃)₃, -CH₂-C₆H₄OCH₂-C₆H₅, -CH₂-O-CH₃, -CH₂-O-C(CH₃)₃, -CH₂-O-Si(CH₃)₃, -CH₂-O-CH₂-C₆H₅, -CH₂-C₆H₄O-Si(CH₃)₃, -CH(CH₃)-O-CH₃, -CH(CH₃)-O-C(CH₃)₃, -CH(CH₃)-O-Si(CH₃)₃, -CH(CH₃) -O-CH₂-C₆H₅, -CH₂-S-C(C₆H₅)₃, -CH₂-S-CH(C₆H₅)₂, -CH₂-S-CH₂-C₆H₅, -CH₂CH₂CH₂CH₂N- (CO)₂C₆H₄,
- R²: H, C₁₋₄-Alkyl - wie CH₃, -CH₂-CH₃-, -CH₂-CH₂-CH₃, -CH(CH₃) -CH₂-CH₃, -CH₂-CH₂-CH₂-CH₃, -CH₂-CH(CH₃)₂, -C(CH₃)₃-, C₂₋₄-Alkenyl - wie -CH₂-CH=CH₂-, C₂₋₄-Alkinyl - wie -CH₂-C≡CH- -, C₆H₅-, -C₆H₄-CH₃,
- R^{s}: Benzyloxycarbonyl (= Z) oder eine sich davon ableitende Schutzgruppe, vorzugsweise Z, DMZ, BZ, CZ, 3CZ, 2CZ, MOZ, NZ, 2NZ und AcOZ (vgl. Houben-Weyl: Methoden der organischen Chemie 4. Auflage Bd. 15/1 Seite 21). Besonders bevorzugt ist Z.

Die erfindungsgemäße Reaktion wird zweckmäßig unter einem inerten Schutzgas wie Helium oder Argon durchgeführt. Besonders vorteilhaft ist der Einsatz von Stickstoff.

Als Lösungsmittel für die Reaktion sind nicht-protische Lösungsmittel geeignet, insbesondere Tetrahydrofuran, 1,2-Dimethoxyethan, Diethoxymethan, Dioxan, Dichlormethan, Trlchlormethan, Tetrachlorkohlenstoff sowie N,N'-Dimethylethylenharnstoff, N,N'-Dimethylpropylenharnstoff und N-Methylpyrrolidon. Vorzugsweise findet 1,2-Dimethoxyethan Verwendung.

Als Basen werden Natrium-t.-butanolat oder Kalium-t.-butanolat verwendet. Vorzugsweise wird Kalium-t-butanolat verwendet. Es werden 2,2 bis 10, vorzugsweise 3,5 bis 6 Äquivalente Base bezogen auf Verbindung II eingesetzt.

Als Alkylierungsmittel wird vorzugsweise Dimethylsulfat verwendet. Es werden 1,2 bis 6, vorzugsweise 2,5-3,5 Äquivalente Alkylierungsmittel bezogen auf Verbindung II eingesetzt.

Dem Reaktionsgemisch wird zweckmäßigerweise ein polarer, protischer Zusatz zugegeben. Diese Zugabe soll frühestens eine Stunde nach beendeter Zugabe des Alkylierungsmittels, jedoch nicht später als 24 Stunden danach erfolgen. Als protische Zusätze eignen sich besonders Wasser und Alkohole, wie Methanol und Ethanol. Der Zusatz erfolgt zweckmäßig in einer Menge von 0,1 - 10, vorzugsweise 1 - 5 Mol-Äquivalenten bezogen auf das Ausgangsmaterial II. Wasser eignet sich besonders gut als protischer Zusatz.

Die Reaktion kann bei Temperaturen zwischen -40°C und +100°C durchgeführt werden, besonders vorteilhaft sind Temperaturen zwischen -10°C und +20°C. Die Aufarbeitung der Reaktionsmischung kann durch Destillation, Extraktion, Kristallisation, Chromatographie oder eine Kombination derselben erfolgen. Bevorzugt wird eine Extraktion nach Ansäuern durchgeführt und anschließend die Verbindung I auskristallisiert.

Zum Ansäuern können Säuren wie Salzsäure, Schwefelsäure, Phosphorsäure, Bromwasserstoffsäure, Essigsäure, Methansulfonsäure verwendet werden. Vorzugsweise wird Schwefelsäure verwendet. Als Extraktionsmittel können mit Wasser nicht mischbare Lösungsmittel wie Pentan, Hexan, Heptan, Octan, Petrolether, Ethylacetat, Diethylether, Di-Isopropylether, Methyl-t.Butylether, Dichlormethan, Chloroform, Tetrachlorkohlenstoff, Benzol, Toluol, Xylol verwendet werden, besonders bevorzugt ist Toluol. Die Kristallisation kann aus organischen Lösungsmitteln wie Pentan, Hexan, Heptan, Octan, Petrolether, Ethylacetat, Dichlormethan, Chloroform, Tetrachlorkohlenstoff, Benzol, Toluol, Xylol, Aceton, Butan-2-on, Methanol, Ethanol, n-Propanol, iso-Propanol, Diethylether, Di-Isopropylether, Methyl-t.Butylether sowie Gemischen derselben erfolgen, bevorzugt werden Toluol sowie Toluol/Heptan-Gemische verwendet.

Enthalten die Aminosäuren weitere reaktive Gruppen, wie beispielsweise Cystein, Serin, Tyrosin, Lysin, Threonin, so müssen diese während der Umsetzung geschützt sein.

Das neue Verfahren besitzt folgende Vorteile:
1. Es können einfach erhältliche Einsatzstoffe mit vorteilhaften Handhabungseigenschaften verwandt werden (Z-Aminosäuren, Dimethylsulfat),
2. es findet keine gleichzeitige Veresterung statt,
3. es liefert hohe Ausbeuten,
4. es wird ein Produkt hoher Reinheit isoliert, welches ohne weitere Reinigung für Folgereaktionen verwandt werden kann,
5. es findet keine Racemisierung statt.

Besonders überraschend ist die positive Auswirkung des protischen Zusatzes auf Reinheit und Ausbeute, weil ein solcher Zusatz erfahrungsgemäß eher zu einem unvollständigen Umsatz, zu Racemisierung und zu Umesterung führt.

### Beispiele

1. (S)-Z-N-Methyl-Phenylalanin
   Zu einer Lösung von 25 g Kalium-t.butanolat in 250 ml Dimethoxyethan wurden bei 0-5°C 14,95 g (S)-Z-Phenylalanin zugegeben. Während 45 min wurden 18,9 g Dimethylsulfat so zugegeben, daß die Temperatur zwischen 0 und 5°C blieb. Anschließend wurde 2 h bei 0 - 5°C nachgerührt, dann wurde 1 h bei 20°C gerührt. Eine HPLC-Analyse zeigt ein Verhältnis von 1 : 2,4 zwischen Edukt und Produkt. Es wurden 2,5 ml Wasser zugegeben, gefolgt von 8,3 g Kalium-t.butanolat (in 75 ml Dimethoxyethan). Während 15 min wurden 6,3 g Dimethylsulfat bei 0 - 5°C zudosiert. Es wurde 2 h bei dieser Temperatur nachgerührt, dann wurde 2 h bei 20°C gerührt. Eine HPLC-Analyse zeigte ein Verhältnis 1 : 69,2 zwischen Edukt und Produkt. Durch Zugabe von 500 ml Wasser wurde die Reaktion abgebrochen. Die organische Phase wurde abgetrennt und die wäßrige Phase mit 300 ml Toluol extrahiert. Die vereinigten organischen Phasen wurden verworfen. Die wäßrige Phase wurde angesäuert und mit Toluol extrahiert. Die Toluol-Phase wurde zur Trockene eingeengt. Das so erhaltene Rohprodukt wurde zweimal aus Toluol umkristallisiert. Es wurden 13 g Produkt mit einer Reinheit von > 95 % isoliert, Fp.: 66 - 67°C, [α]_{D} = -59,5° (c = 1,0 in CH₂Cl₂).
   Analog Beispiel 1 wurden hergestellt:
2. (S)-Z-N-Methyl-Valin
   Ausbeute: 85 %, Fp.: 69 - 71°C, [α]_{D} = -84,6° (c = 2,0 in C₂H₅OH)
   Bei der Herstellung dieser Verbindung analog Beispiel 1, jedoch ohne Zusatz von Wasser vor der Zugabe des Kalium-t.-butanolats betrug die Ausbeute 61 %.
3. (+)-Z-N-Methyl-Isoleucin
   Ausbeute: 80 %, Fp.: 55-56°C.
   Bei der Herstellung dieser Verbindung analog Beispiel 1, jedoch ohne Zusatz von Wasser vor der Zugabe des Kalium-t.-butanolats betrug die Ausbeute 65 %.
4. (S)-Z-N-Methyl-O-t.butyl-Serin
5. (S)-Z-N-Ethyl-Phenylalanin
6. (S)-Z-N-Ethyl-Valin
   Analog lassen sich herstellen:
   (S)-Z-N-Methyl-Glycin, (S)-Z-N-Methyl-Alanin, (S)-Z-N-Methyl-Cystein-(S-Benzyl); (S)-Z-N-Methyl-Leucin, (S)-Z-N-Methyl-Methionin, (S)-Z-N-Methyl-Threonin-(O-Benzyl).

## Patentansprüche

1. Verfahren zur Herstellung N-geschützter-N-alkylierter Aminosäuren der Formel I: 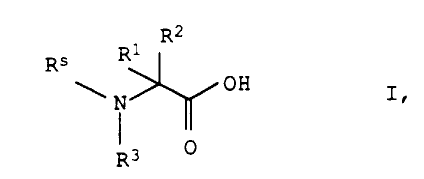 worin
R^{s} für eine in der Peptidsynthese übliche Schutzgruppe steht,
R¹ die Seitenkette einer proteinogenen Aminosäure oder gegebenenfalls ein funktionelles Derivat derselben bedeutet,
R² für Wasserstoff, C₁₋₆-Alkyl, C₂₋₆-Alkenyl, C₂₋₆-Alkinyl oder gegebenenfalls C₁₋₄-alkylsubstituiertes Phenyl oder Benzyl steht und
R³ Methyl oder Ethyl ist,
dadurch gekennzeichnet, daß man eine Verbindung der Formel II: 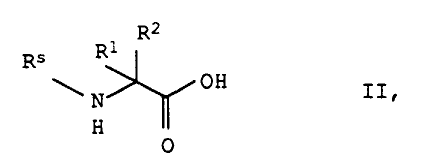 worin R¹, R² und R^{s} die oben angegebene Bedeutung haben, zu einer Lösung von Natrium- oder Kalium-tert.-butanolat in einem nichtprotischen organischen Lösungsmittel gibt und anschließend mit Dimethyl- oder Diethylsulfat versetzt.

2. Verfahren nach Anspruch 1, dadurch gekennzeichnet, daß als Base Kalium-tert.butanolat und als Alkylierungsmittel Dimethylsulfat verwendet wird.

3. Verfahren nach Anspruch 1, dadurch gekennzeichnet, daß als Base Kalium-tert.-butanolat und als Alkylierungsmittel Dimethylsulfat sowie Wasser als protischer Zusatz verwendet wird.

4. Verfahren nach Anspruch 1, dadurch gekennzeichnet, daß als Schutzgruppe R^{s} ein gegebenenfalls substituierter Benzyloxycarbonylrest verwendet wird.

## Claims

1. A process for preparing N-protected N-alkylated amino acids of the formula I 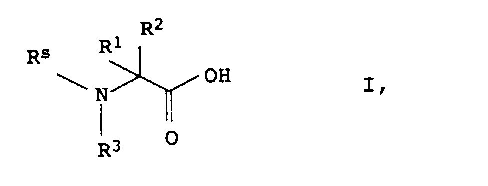 where
R^{s} is a conventional protective group for peptide synthesis,
R¹ is the side chain of a proteinogenous amino acid or a functional derivative thereof,
R² is hydrogen, C₁₋₆-alkyl, C₂₋₆-alkenyl, C₂₋₆-alkynyl or unsubstituted or C₁₋₄-alkyl-substituted phenyl or benzyl, and
R³ is methyl or ethyl,
which comprises adding a compound of the formula II: 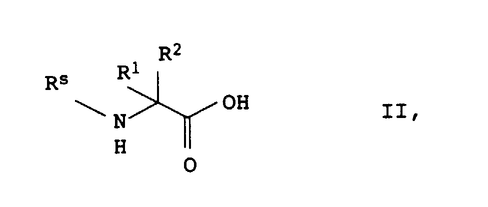 where R¹, R² and R^{s} have the abovementioned meanings, to a solution of sodium or potassium tert-butanolate in a non-protic organic solvent and subsequently adding dimethyl or diethyl sulfate.

2. A process as claimed in claim 1, wherein potassium tert-butanolate is used as base and dimethyl sulfate is used as alkylating agent.

3. A process as claimed in claim 1, wherein potassium tert-butanolate is used as base and dimethyl sulfate is used as alkylating agent, and water is used as protic additive.

4. A process as claimed in claim 1, wherein an unsubstituted or substituted benzyloxycarbonyl radical is used as protective group R^{s}.

## Revendications

1. Procédé de préparation d'aminoacides N-alkylés, N-protégés, répondant à la formule I : 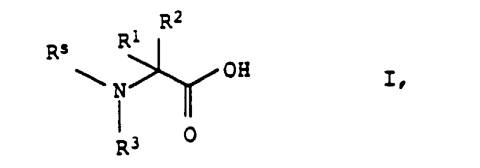 dans laquelle
R^{s} représente un radical protecteur usuel utilisé pour la synthèse des peptides,
R¹ représente la chaîne latérale d'un aminoacide protéinogène, ou éventuellement un dérivé fonctionnel de celui-ci,
R² représente un atome d'hydrogène, un radical alkyle en C₁ à C₆, alcényle en C₂ à C₆, alcynyle en C₂ à C₆, ou un radical benzyle ou phényle à substitution alkylique en C₁ à C₄, éventuelle et
R^{s} représente le radical méthyle ou éthyle
caractérisé en ce que l'on ajoute un composé de la formule II : 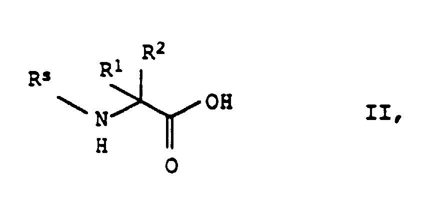 dans laquelle R¹, R² R^{s} possèdent les significations qui leur ont été précédemment attribuées, à une solution de tert-butanolate de sodium ou de potassium dans un solvant organique aprotique et on y ajoute ensuite du sulfate de diméthyle ou de diéthyle.

2. Procédé suivant la revendication 1, caractérisé en ce que l'on utilise le tert-butanolate de potassium, à titre de base et le sulfate de diméthyle, à titre d'agent d'alkylation.

3. Procédé suivant la revendication 1, caractérisé en ce que l'on utilise le tert-butanolate de potassium, à titre de base et le sulfate de diméthyle, à titre d'agent d'alkylation, ainsi que de l'eau, à titre d'additif protique.

4. Procédé suivant la revendication 1, caractérisé en ce que l'on utilise, à titre de groupe protecteur R^{s}, un reste benzyloxycarbonyle éventuellement substitué.
